# EUROPEAN PATENT SPECIFICATION

(11) **EP 3 174 469 B1**
(45) Date of publication and mention of the grant of the patent: **29.01.2020**
(21) Application number: 15749916.1
(22) Date of filing: 30.07.2015
(51) Int. Cl.: A61B 10/02, A61B 10/04, A61B 17/00

(54) **SCOOPER CORE NEEDLE**
KERNNADEL MIT SCHAUFEL
AIGUILLE CREUSE DE RAMASSAGE

(30) Priority: 31.07.2014 US 201462031629 P
(43) Date of publication of application: 07.06.2017
(73) Proprietor: Boston Scientific Scimed, Inc., Maple Grove, MN 55311-1566 (US)
(72) Inventor: FAULKNER, Daniel, Cambridge, Massachusetts 02138 (US); LAJOIE, Kimberly, Seattle, Washington 98107 (US); BUDIMAN, Tantra, Newton, Massachusetts 02459 (US); WONG, Eric, Framingham, Massachusetts 01701 (US); SMITH, Paul, Smithfield, Rhode Island 02917 (US); ALMEIDA, Brian, Newton, Massachusetts 02459 (US); SHAH, Vishal, Whitehall, Pennsylvania 18052 (US); ELFMAN, Justin, Cherry Hill, New Jersey 08003 (US); DEVRIES, Robert B., Northborough, Massachusetts 01532 (US)
(74) Representative: Vossius & Partner Patentanwälte Rechtsanwälte mbB
(86) International application number: PCT/US2015/042809
(87) International publication number: WO 2016/019097

(56) References cited:
- WO-A1-89/05608
- WO-A1-2012/024227
- WO-A2-94/15533
- WO-A2-2008/027829
- US-A1- 2013 197 551
- US-B1- 6 203 524

## Description

### Background

Needle biopsy procedures may be used for the diagnosis and the staging of disease. For example, a fine aspiration needle may be advanced through a working channel of an endoscope to a target tissue site. Although fine needle aspiration may be a highly sensitive and specific procedure, it may be difficult to acquire a suitable sample under certain clinical situations. The more cells or tissue that can be acquired, the greater the potential for a definitive diagnosis. Larger gauge needles, however, may be difficult to pass along tortuous paths through anatomy to target sites and may acquire samples including more blood, making it more difficult to obtain a diagnosis.

In document WO 2008/027829 A2 apparatuses and methods for performing minimally invasive medical procedures are disclosed. In one embodiment, an apparatus includes an outer body and an inner body movably disposable within a lumen of the outer body. At least one of the inner body or the outer body is configured to be moved relative to the other while inserted into the tissue such that a cutting portion severs at least a portion of the tissue and the severed portion of the tissue is disposed within a lumen of the inner body.

Also, in US 2013/197551 A1 a tissue removal device including customizable tips is disclosed. An aspect of said disclosure may include a flexible cutting blade extending from a threaded shaft.

### Summary

The present disclosure is directed to a device for collecting a tissue sample, comprising a needle extending longitudinally from a proximal end to a distal end and comprising a lumen extending therethrough and a stylet movably housed in the lumen of the needle, the stylet extending along a longitudinal axis from a proximal end to a distal end and comprising a scooper at the distal end thereof, the scooper connected to a proximal portion of the stylet via a hinge biased toward a bent position in which the scooper is bent away from the longitudinal axis, the stylet movable between a constrained configuration, in which an interior surface of the needle constrains the scooper in axial alignment with the proximal portion, and the tissue-collecting configuration, in which the scooper extends distally past the distal end of the needle such that the hinge is permitted to revert to the biased bent position so that the scooper bends away from the longitudinal axis, the scooper including a channel extending longitudinally therethrough and a window extending laterally through a wall thereof in communication with the channel to collect a tissue sample therethrough as the stylet is moved from the tissue-collecting configuration to the constrained configuration.

The distal end of the stylet may be at least partially closed.

The distal end of the stylet may include a tapered tip.

The hinge may be a living hinge.

The needle may include a longitudinal slot extending proximally from the distal end thereof, the longitudinal slot sized and shaped to pass a portion of the hinge therethrough, when the stylet is in the tissue-collecting configuration.

The stylet may be rotatable relative to the needle between a first position and a second position in the constrained configuration, the window may be substantially aligned with the longitudinal slot in the first position and substantially opposing the longitudinally slot in the second position.

The needle may be sized and shaped to be inserted through a working channel of an endoscope.

The present disclosure is also directed to a device for collecting a tissue sample, comprising a needle extending longitudinally from a proximal end to a distal end and comprising a lumen extending therethrough and a stylet movably housed in the lumen of the needle, the stylet comprising a proximal portion extending along a longitudinal axis and a scooper connected to a distal end of the proximal portion via a hinge biased toward a bent position such that the stylet is movable between a constrained configuration, in which the scooper is constrained along the longitudinal axis via an interior surface of the lumen of the needle, and a tissue-collecting configuration in which the scooper extends distally past the distal end of the needle such that the hinge reverts to the biased bent position bending the scooper away from the longitudinal axis, the scooper including a channel extending therethrough and a window extending laterally through a wall thereof in communication with the channel.

The distal end of the stylet may be at least partially closed.

The distal end of the stylet may include a tapered tip.

The hinge may be a living hinge.

The needle may include a longitudinal slot extending proximally from the distal end thereof, the longitudinal slot sized and shaped to pass a portion of the hinge therethrough, when the stylet is in the tissue-collecting configuration.

The stylet may rotatable relative to the needle between a first position and a second position in the constrained configuration, the window may be substantially aligned with the longitudinal slot in the first position and substantially opposing the longitudinally slot in the second position.

The needle may be sized and shaped to be inserted through a working channel of an endoscope.

The needle and stylet may be formed of a material sufficiently flexible to permit insertion thereof through even tortuous paths of a patient body.

The present disclosure is also directed to a method for collecting a tissue sample, comprising inserting a needle device, in a constrained position, into a target tissue via a working channel of an endoscope, the device including a needle and a stylet housed therewithin, the stylet including a scooper constrained via an interior surface of the needle in axial alignment with a proximal portion of the stylet in the constrained configuration, moving the stylet distally relative to the needle such that the scooper reverts to a biased tissue-collecting configuration in which the scooper is bent away from a longitudinal axis of the device, and moving the needle distally over the scooper such that a tissue sample is collected within the channel via the window as the scooper is moved toward the constrained configuration.

### Brief Description

Fig. 1 shows a longitudinal side view of a device according to an exemplary embodiment of the present disclosure, in a first position;
Fig. 2 shows a longitudinal side view of the device of Fig. 1, in a second position;
Fig. 3 shows a longitudinal side view of the device of Fig. 1, in a third position;
Fig. 4 shows a longitudinal side view of a needle of the device of Fig. 1; and
Fig. 5 shows a longitudinal side view of a stylet of the device of Fig. 1.

### Detailed Description

The present disclosure may be further understood with reference to the following description and the appended drawings, wherein like elements are referred to with the same reference numerals. The present disclosure relates to endoscopic devices and, in particular, devices for obtaining tissue samples. Exemplary embodiments of the present disclosure describe a device comprising a stylet including a scooper at a distal end thereof and a needle longitudinally movable relative to the stylet to move the device between a constrained configuration and a tissue-collecting configuration. In the tissue-collecting configuration, the scooper extends distally from a distal end of the needle to bend or rotate away from a longitudinal axis of the stylet. Thus, moving the needle distally over the scooper permits tissue to be collected through a window of the scooper as the device is moved to the constrained configuration. It will be understood by those of skill in the art that the device is not restricted by a diameter of the needle and thus collects a larger tissue sample than would be possible with traditional biopsy needles. It should be noted that the terms "proximal" and "distal" as used herein, are intended to refer to a direction toward (proximal) and away from (distal) a user of the device.

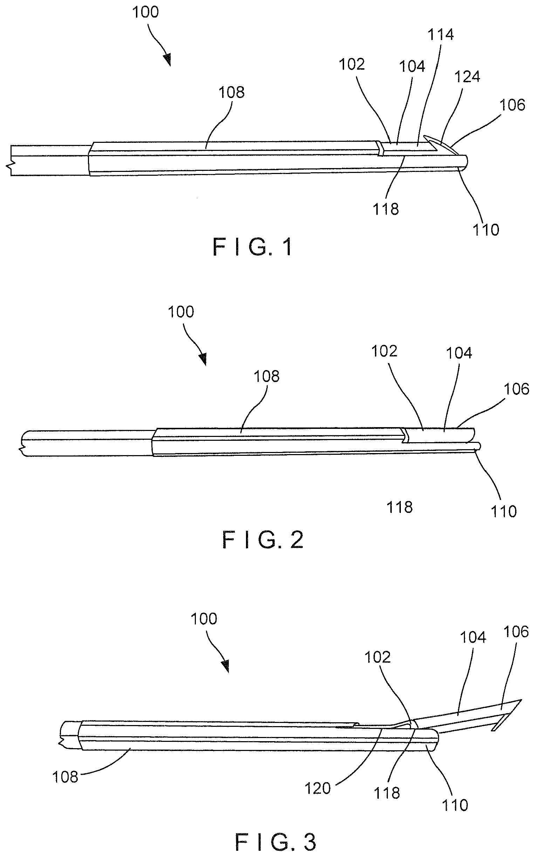

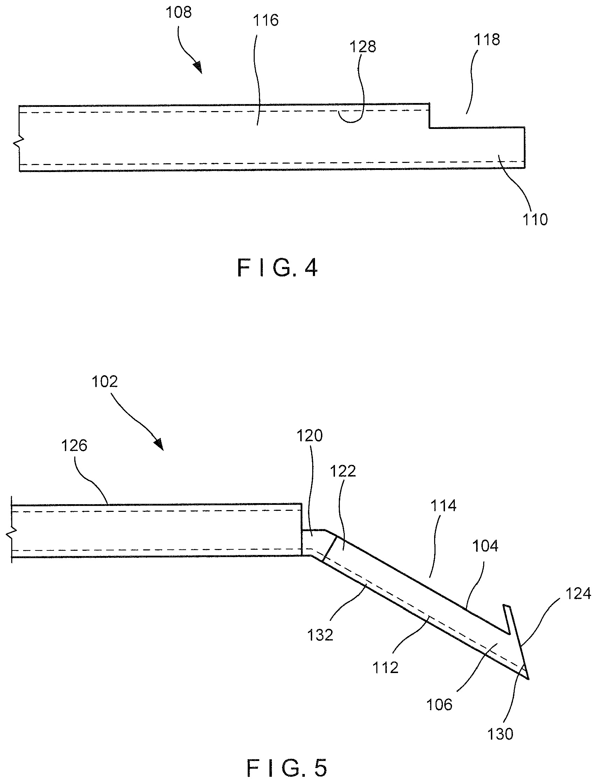

As shown in Figs. 1 - 5, a device 100 according to an exemplary embodiment of the present disclosure comprises a stylet 102 including a scooper 104 at a distal end 106 thereof and a needle 108 longitudinally movable over the stylet 102 to move the device 100 between a constrained configuration and a tissue-collecting configuration. The scooper 104 includes a channel 112 extending longitudinally therethrough and a window 114 extending laterally through a wall thereof in communication with the channel 112 such that target tissue may be collected in the channel 112 via the window 114. The scooper 104 is connected to a proximal portion 126 of the stylet 102 via a hinge 120 biasing the scooper 104 toward a tissue-collecting configuration in which the scooper 104 is bent or rotated away from a longitudinal axis of the proximal portion of the stylet 102. The scooper 104 includes a channel 112 extending longitudinally therethrough and a window 114 extending laterally through a wall 132 thereof in communication with the channel 112 such that, when the scooper 104 is in the tissue-collecting configuration, the window 114 faces the longitudinal axis of the stylet 102. In the constrained configuration, the needle 108 extends over the scooper 104 such that an interior surface 128 thereof constrains the scooper 104, holding the scooper 104 in axial alignment with the proximal portion of the stylet 102. In the tissue-collecting configuration, the stylet 102 is moved distally relative to the needle 108 such that the scooper 104 extends distally past a distal end 110 of the needle 108 and is permitted to revert to its biased position in which the scooper 104 is angled with respect to the proximal portion of the stylet 102. The device 100 may be inserted to a target site in a living body (e.g., through a body lumen accessed via a natural body orifice) through, for example, a working channel of an endoscope or other insertion instrument. The device 100 is advanced to a site adjacent to target tissue while in the constrained configuration. When the target site has been reached, the distal end 110 is inserted into the target tissue and the stylet 102 is moved distally with respect to the needle 108 such that the scooper 104 reverts to the biased tissue-collecting configuration. The needle 108 is then moved distally over the scooper 104 to draw the scooper 104 back into alignment with the axis of the needle 108 collecting a tissue sample via the window 114 in the channel 112, as the device 100 is moved to the constrained configuration. It will be understood by those of skill in the art that the scooper 104 enables collection of a larger tissue sample than would be possible with traditional biopsy needles.

The needle 108 extends longitudinally from a proximal end to the distal end 110 formed, for example, as a tissue piercing distal tip. The needle 108 includes a lumen 116 extending therethrough and a lateral cross-sectional area of the needle 108 is sized and shaped to be inserted through a working channel of an insertion device such as a flexible endoscope. The needle 108 of this embodiment is formed of a material sufficiently flexible to enable insertion of the device 100 through even tortuous paths of the body such as those traversed by flexible endoscopes. The distal end 110 may include a longitudinal slot 118 extending proximally therefrom. The longitudinal slot 118 is sized and shaped to permit a portion of the hinge 120 and/or a proximal end 122 of scooper 104 to extend therethrough, when the device 100 is in the tissue-collecting position. It will be understood by those of skill in the art, however, that the longitudinal slot 118 is not required, and the stylet 102 may simply be moved further distally with respect to the needle 108 to permit the stylet 102 to revert to the biased tissue-collecting configuration.

The stylet 102 extends longitudinally from a proximal end to a distal end 124 and includes the channel 112 extending through at least a distal portion (including the scooper 104) thereof. The distal end 124, however, is closed such that the distal end 124 may be used to pierce tissue as the device 100 is being inserted into the target tissue. Thus, the distal end 124 may include a tapered tip 130 to facilitate the piercing of tissue. The distal end 124 is not required to be fully closed, however, and may be partially closed to facilitate insertion into the target tissue while also retaining any portion of tissue received in the channel 112 via the window 114. The window 114 extends laterally through a wall 132 of the scooper 104, from the closed distal end 124 to the proximal end 122 of the scooper 104. The scooper 104 is connected to the proximal portion 126 of the stylet 102 via the hinge 120. The hinge 120 may, for example, be a living hinge biased toward a bent position such that the stylet 102 is moved toward the tissue-collecting configuration with the scooper 104 angled relative to the longitudinal axis of the stylet 102 when unconstrained. As described above, the window 114 passes through a portion of the scooper 104 which, when the stylet 102 is in the tissue-collecting configuration, faces toward the longitudinal axis of the stylet 102. Thus, as the scooper 104 is moved toward the longitudinal axis into axial alignment with the proximal portion 126 in the constrained configuration, the tissue sample is "scooped" or collected into the channel 112 via the window 114. The stylet 102 is moved to the constrained configuration by moving the needle 108 distally over the scooper 104, trapping the collected tissue sample in the channel 112 via an interior surface 128 of the lumen 116 of the needle 108, which extends over the window 114. Although the hinge 120 is described and shown as a living hinge, it will be understood by those of skill in the art that the hinge 120 may take any of a variety of forms so long as the scooper 104 is biased away from the longitudinal axis of the stylet 102.

According to an exemplary surgical method utilizing the device 100, the device 100 is inserted into target tissue within a living body via a working channel of an endoscope or other insertion device, in the constrained configuration. The device 100 may be inserted into the body in a first position of the constrained configuration, as shown in Fig .1, in which the window 114 of the scooper 104 is substantially aligned with the longitudinal slot 118 of the needle 108. The tapered tip 130 may extend slightly distally of the distal end 110 of the needle 108 to facilitate piercing of the target tissue as the device 100 is inserted thereinto. Once the target tissue has been pierced, the stylet 102 is rotated substantially 180 degrees about the longitudinal axis thereof to a second position of the constrained configuration, as shown in Fig. 2, such that the window 114 substantially opposes the longitudinal slot 118 of the needle 108 and is covered by a portion of the interior surface 128 of the needle 108. The stylet 102 is then moved distally with respect to the needle 108 such that the stylet 102 is permitted to revert to the biased tissue-collecting configuration in which the scooper 104 is bent or angled away from the longitudinal axis, as shown in Fig. 3. As discussed above, the hinge 120 and/or the proximal end 122 of the scooper 104 extends through the longitudinal slot 118 as the stylet 102 is moved to the tissue-collecting configuration. Finally, the needle 108 is moved distally over the scooper 104 such that the scooper 104 collects a tissue sample within the channel 112 via the window 114, as the stylet 102 is moved to the constrained configuration. The tissue sample is trapped within the channel 112 via a portion of the interior surface 128 of the lumen 116 of the needle 108 covering the window 114.

Although the exemplary surgical method describes moving the stylet 102 relative to the needle 108 between first and second positions of the constrained configuration, it will be understood by those of skill in the art that the device 100 may be utilized without rotating the stylet 102 relative to the needle 108. In particular, the device 100 may be inserted into the target tissue, in the constrained position, with the window 114 opposing the longitudinal slot 118, so that the stylet 102 and the needle 108 may simply be moved longitudinally relative to one another between the constrained and tissue-collecting configurations. In addition, for embodiments of the device 100 in which the needle 108 does not include the longitudinal slot 118, a rotational orientation of the stylet 102 relative to the needle 108 will have no bearing on the use of the device 100. It will also be understood by those of skill in the art that the stylet 102 and the needle 108 may be repeatedly moved relative to one another between the constrained and tissue-collecting configurations until a desired tissue sample has been collected.

It will be apparent to those skilled in the art that variations can be made in the structure and methodology of the present disclosure, without departing from the scope of the disclosure. Thus, it is intended that the present disclosure cover the modifications and variations of this disclosure provided that they come within the scope of the appended claims and their equivalents.

## Claims

1. A device (100) for collecting a tissue sample, comprising:
a needle (108) extending longitudinally from a proximal end to a distal end (110) and comprising a lumen (116) extending therethrough; and
a stylet (102) movably housed in the lumen (116) of the needle (108), the stylet (102) extending along a longitudinal axis from a proximal end (126) to a distal end (106) and comprising a scooper (104) at the distal end (106) thereof, the scooper (104) connected to a proximal portion of the stylet (102) via a hinge (120) biased toward a bent position in which the scooper (104) is bent away from the longitudinal axis, the stylet (102) movable between a constrained configuration, in which an interior surface (128) of the needle (108) constrains the scooper (104) in axial alignment with the proximal portion, and the tissue-collecting configuration, in which the scooper (104) extends distally past the distal end (110) of the needle (108) such that the hinge (120) is permitted to revert to the biased bent position so that the scooper (104) bends away from the longitudinal axis, the scooper (104) including a channel (112) extending longitudinally therethrough and a window (114) extending laterally through a wall (132) thereof in communication with the channel (112) to collect a tissue sample therethrough as the stylet (102) is moved from the tissue-collecting configuration to the constrained configuration;
**characterized in that** the needle (108) includes a longitudinal slot (118) extending proximally from the distal end (110) thereof, the longitudinal slot (118) sized and shaped to pass a portion of the hinge (120) therethrough, when the stylet (102) is in the tissue-collecting configuration.

2. The device (100) of claim 1, wherein the distal end (106) of the stylet (102) is at least partially closed.

3. The device (100) of claim 2, wherein the distal end (106) of the stylet (102) includes a tapered tip.

4. The device (100) of any one of claims 1 to 3, wherein the hinge (120) is a living hinge.

5. The device (100) of any one of claims 1-4, wherein the stylet (102) is rotatable relative to the needle (108) between a first position and a second position in the constrained configuration, the window (114) being substantially aligned with the longitudinal slot (118) in the first position and substantially opposing the longitudinally slot in the second position.

6. The device (100) of any one of claims 1 to 5, wherein the needle (108) is sized and shaped to be inserted through a working channel of an endoscope.

7. The device (100) of any one of claims 1 to 6, wherein the needle (108) and stylet (102) are formed of a material sufficiently flexible to permit insertion thereof through even tortuous paths of a patient body.

## Patentansprüche

1. Vorrichtung (100) zur Entnahme einer Gewebeprobe, aufweisend:
eine Nadel (108), die sich in Längsrichtung von einem proximalen Ende zu einem distalen Ende (110) erstreckt und ein sich durch sie erstreckendes Lumen (116) aufweist; und
einen Stab (102), der beweglich im Lumen (116) der Nadel (108) untergebracht ist, wobei sich der Stab (102) entlang einer Längsachse von einem proximalen Ende (126) zu einem distalen Ende (106) erstreckt und an seinem distalen Ende (106) eine Schaufel (104) aufweist, wobei die Schaufel (104) über ein Scharnier (120) mit einem proximalen Abschnitt des Stabs (102) in eine gebogene Stellung vorgespannt verbunden ist, in der die Schaufel (104) von der Längsachse weggebogen ist, wobei der Stab (102) zwischen einer eingespannten Konfiguration, in der eine Innenfläche (128) der Nadel (108) die Schaufel (104) in axialer Ausrichtung zum proximalen Abschnitt einspannt, und der Gewebeentnahmekonfiguration, in der sich die Schaufel (104) distal am distalen Ende (110) der Nadel (108) vorbei erstreckt, so dass das Scharnier (120) in die vorgespannte gebogene Stellung zurückkehren kann und sich die Schaufel (104) von der Längsachse wegbiegt, wobei die Schaufel (104) einen Kanal (112), der sich in Längsrichtung durch sie hindurch erstreckt, und ein sich seitlich durch eine ihrer Wände (132) erstreckendes Fenster (114) umfasst, das mit dem Kanal (112) in Verbindung steht, um durch diesen eine Gewebeprobe zu entnehmen, während der Stab (102) von der Gewebeentnahmekonfiguration in die eingespannte Konfiguration bewegt wird;
**dadurch gekennzeichnet, dass**
die Nadel (108) einen länglichen Schlitz (118) umfasst, der sich proximal von deren distalen Ende (110) aus erstreckt, wobei der längliche Schlitz (118) bemessen und geformt ist, um einen Abschnitt des Scharniers (120) dadurch hindurch zu führen, wenn sich der Stab (102) in der Gewebeentnahmekonfiguration befindet.

2. Vorrichtung (100) nach Anspruch 1, wobei das distale Ende (106) des Stabs (102) mindestens teilweise geschlossen ist.

3. Vorrichtung (100) nach Anspruch 2, wobei das distale Ende (106) des Stabs (102) eine konische Spitze umfasst.

4. Vorrichtung (100) nach einem der Ansprüche 1 bis 3, wobei das Scharnier (120) ein Filmscharnier ist.

5. Vorrichtung (100) nach einem der Ansprüche 1 bis 4, wobei der Stab (102) in der eingespannten Konfiguration in Bezug auf die Nadel (108) zwischen einer ersten Stellung und einer zweiten Stellung drehbar ist, wobei das Fenster (114) in der ersten Stellung im Wesentlichen mit dem länglichen Schlitz (118) fluchtet und in der zweiten Stellung dem länglichen Schlitz im Wesentlichen gegenüberliegt.

6. Vorrichtung (100) nach einem der Ansprüche 1 bis 5, wobei die Nadel (108) so bemessen und geformt ist, dass sie durch einen Arbeitskanal eines Endoskops eingeführt werden kann.

7. Vorrichtung (100) nach einem der Ansprüche 1 bis 6, wobei die Nadel (108) und der Stab (102) aus einem ausreichend flexiblen Material gebildet sind, um eine Einführung auch durch gewundene Wege des Körpers eines Patienten zu gestatten.

## Revendications

1. Dispositif (100) pour collecter un échantillon de tissu, comprenant :
une aiguille (108) s'étendant longitudinalement d'une extrémité proximale à une extrémité distale (110) et comprenant une lumière (116) s'étendant à travers cette dernière ; et
un stylet (102) logé, de manière mobile, dans la lumière (116) de l'aiguille (108), le stylet (102) s'étendant le long d'un axe longitudinal d'une extrémité proximale (126) à une extrémité distale (106) et comprenant un dispositif de prélèvement (104) au niveau de son extrémité distale (106), le dispositif de prélèvement (104) étant raccordé à une partie proximale du stylet (102) via une charnière (120) sollicitée vers une position pliée dans laquelle le dispositif de prélèvement (104) est plié à distance de l'axe longitudinal, le stylet (102) étant mobile entre une configuration contrainte dans laquelle une surface intérieure (128) de l'aiguille (108) contraint le dispositif de prélèvement (104) en alignement axial avec la partie proximale, et la configuration de collecte de tissu dans laquelle le dispositif de prélèvement (104) s'étend de manière distale au-delà de l'extrémité distale (110) de l'aiguille (108) de sorte que la charnière (120) est autorisée à se retourner dans la position pliée sollicitée de sorte que le dispositif de prélèvement (104) se plie à distance de l'axe longitudinal, le dispositif de prélèvement (104) comprenant un canal (112) s'étendant longitudinalement à travers ce dernier et une fenêtre (114) s'étendant latéralement à travers une de ces parois (132) en communication avec le canal (112) pour collecter un échantillon de tissu à travers cette dernière lorsque le stylet (102) passe de la configuration de collecte de tissu à la configuration contrainte ;
**caractérisé en ce que** :
l'aiguille (108) comprend une fente longitudinale (118) s'étendant de manière proximale à partir de son extrémité distale (110), la fente longitudinale (118) étant dimensionnée et formée pour faire passer une partie de la charnière (120) à travers cette dernière, lorsque le stylet (102) est dans la configuration de collecte de tissu.

2. Dispositif (100) selon la revendication 1, dans lequel l'extrémité distale (106) du stylet (102) est au moins partiellement fermée.

3. Dispositif (100) selon la revendication 2, dans lequel l'extrémité distale (106) du stylet (102) comprend une pointe progressivement rétrécie.

4. Dispositif (100) selon l'une quelconque des revendications 1 à 3, dans lequel la charnière (120) est une charnière active.

5. Dispositif (100) selon l'une quelconque des revendications 1 à 4, dans lequel le stylet (102) peut tourner par rapport à l'aiguille (108) entre une première position et une seconde position dans la configuration contrainte, la fenêtre (114) étant sensiblement alignée avec la fente longitudinale (118) dans la première position et sensiblement opposée à la fente longitudinale dans la seconde position.

6. Dispositif (100) selon l'une quelconque des revendications 1 à 5, dans lequel l'aiguille (108) est dimensionnée et formée pour être insérée à travers un canal de travail d'un endoscope.

7. Dispositif (100) selon l'une quelconque des revendications 1 à 6, dans lequel l'aiguille (108) et le stylet (102) sont formés avec un matériau suffisament flexible pour permettre son insertion même à travers des trajectoires tortueuses du corps d'un patient.
